# EUROPÄISCHE PATENTSCHRIFT

(11) **EP 1 312 322 B1**
(45) Veröffentlichungstag und Bekanntmachung des Hinweises auf die Patenterteilung: **29.09.2004**
(21) Anmeldenummer: 01811111.2
(22) Anmeldetag: 20.11.2001
(51) Int. Cl.: A61F 2/34, A61F 2/30

(54) **Künstliche Gelenkpfanne**
Artificial Joint Cup
Cotyle artificiel

(43) Veröffentlichungstag der Anmeldung: 21.05.2003
(73) Patentinhaber: Centerpulse Orthopedics Ltd., 6340 Baar (CH)
(72) Erfinder: Löhr, Jochen, Prof. Dr., 8044 Zürich (CH); Willi, Roland, 8413 Neftenbach (CH)
(74) Vertreter: Manitz, Finsterwald & Partner GbR

(56) Entgegenhaltungen:
- EP-A- 0 563 503
- EP-A- 1 082 949
- US-A- 4 936 856
- US-A- 5 507 825
- US-A- 5 624 464

## Beschreibung

Die Erfindung betrifft eine künstliche Hüftgelenkpfanne gemäss dem Oberbegriff des unabhängigen Anspruchs 1 oder 2.

Aus der EP 0 563 503 ist eine Stütz- oder Aussenschale für eine künstliche Hüftgelenkpfanne bekannt, die in ihrer Grundform als Halbkugel ausgebildet ist und in deren Polbereich eine Kalotte abgeschnitten ist, so dass die Schale dort eine Öffnung aufweist. In der Schalenfläche sind Durchtrittsöffnungen für Knochenschrauben zur Fixierung im Beckenknochen vorgesehen. Auf der Innenseite der Aussenschale sind drei Noppen angebracht, die eine einzementierbare Innenschale so auf Distanz halten, dass zwischen den beiden Schalen ein Knochenzementbett konstanter Dicke erzeugt wird, in dem die Noppenhöhe dem zu erzeugenden Spalt entspricht. Beim Implantieren der bekannten Hüftgelenkpfanne wird die Aussenschale mit Knochenschrauben im Beckenknochen verankert. Anschliessend wird ein Knochenzement appliziert, der den Zwischenraum zwischen der Aussenschale und dem Knochengewebe füllt und in der Aussenschale ein Zementbett bildet, so dass eine Innenschale positionierbar ist und nach dem Aushärten des Knochenzements gehalten wird.

Der Nachteil einer solchen Hüftgelenkpfanne, die die bekannte Aussenschale und eine entsprechende Innenschale umfasst, besteht darin, dass nachdem die Aussenschale im Beckenknochen fixiert ist, für die Positionierung der Innenschale bestimmte notwendige Freiheitsgrade nicht mehr verfügbar sind. Das heisst, nachdem die Position der Aussenschale festgelegt ist, ist eine gezielte Verschiebung des Drehzentrums des Gelenks zur Anpassung an den Bandapparat praktisch nicht mehr möglich.

Die Aufgabe der Erfindung ist es daher, eine zweiteilige künstliche Hüftgelenkpfanne vorzuschlagen, die eine äussere Verankerungsschale mit Verankerungshilfen zur Fixierung in einer Knochenhöhlung und eine Innenschale umfasst, wobei die Verankerungsschale so ausgestaltet ist, dass die Innenschale in Bezug auf die Verankerungsschale entlang einer vorgebbaren Kurve so positionierbar ist, dass eine gezielte Rekonstruktion eines Drehzentrums eines Gelenkes vor der definitiven Verankerung der Innenschale noch möglich ist.

Die diese Aufgabe lösenden Gegenstände der Erfindung sind durch die Merkmale des unabhängigen Anspruchs 1 oder 2 gekennzeichnet. Die jeweiligen abhängigen Ansprüche beziehen sich auf besonders vorteilhafte Ausführungsformen der Erfindung. Die Primärverankerung der erfindungsgemässen künstlichen Gelenkpfanne erfolgt in einer präparierten Knochenhöhlung an Verankerungshilfen, die die Schalenfläche der äusseren Verankerungsschale aufweist, mit zusätzlichen Befestigungselementen, wie zum Beispiel mit Knochenschrauben. Vor dem Befestigen der Verankerungsschale in der Knochenhöhlung wird eine Lasche durch plastische Deformation dem Verlauf des Knochens ausserhalb der Knochenhöhlung angepasst, so dass die Lasche eine Auflage auf dem Knochen findet und so zusätzlich zur Verankerung der künstlichen Gelenkpfanne beiträgt.

Ein besonderer Vorteil der erfindungsgemässen künstlichen Gelenkpfanne ist darin zu sehen, dass auch nach der Positionierung der Verankerungsschale in der Knochenhöhlung die Lage der Innenschale in Bezug auf die Verankerungsschale entlang einer vorgebbaren Kurve variabel festlegbar ist. Dadurch ist das Drehzentrum eines Gelenkes weitgehend unabhängig von der Position der Verankerungsschale in der Knochenhöhlung rekonstruierbar.

In einer besonders vorteilhaften Ausführungsform der künstlichen Gelenkpfanne ist die äussere Verankerungsschale bezüglich einer Symmetrieachse längsoval ausgeführt, wobei in einem Quadranten der Schale ein sektorförmiger Abschnitt fehlt, so dass die Verankerungsschale in Richtung der Symmetrieachse halb offen ist. Unterhalb einer Äquatoriallinie der Schale befindet sich in jeder Schalenhälfte je ein als Sollbruchlinie ausgebildeter Schlitz. Dieser Schlitz gestattet es, von der Verankerungsschale einen peripheren Teil von einer der Schalenhälften zu entfernen. Je nachdem in welcher Schalenhälfte der periphere Teile entfernt wird, kann die künstliche Gelenkpfanne in einer linken Körperhälfte des Patienten oder in einer rechten Körperhälfte eingepflanzt werden. Im Polbereich der Verankerungsschale fehlt ein Kugelflächenabschnitt, so dass die Verankerungsschale in diesem Bereich eine Öffnung aufweist, die eine Ebene definiert. Am Rand der Öffnung befinden sich Führungsrippen, die in Bezug zur Symmetrieachse in beiden Schalenhälften angeordnet sind und die Führung einer Innenschale, die zur Aufnahme eines Gelenkkopfes geeignet ist, gestatten. Die Verbindungsstellen zwischen den Führungsrippen und dem Rand der Öffnung sind als Sollbiegelinien ausgebildet. Damit ist durch Biegen der Führungsrippen in vorgegebene Richtungen der senkrechte Abstand, den die Innenschale in Bezug auf die durch die Öffnung definierte Ebene einnimmt, einstellbar. Darüber hinaus kann die endgültige Position der Innenschale in Bezug auf die Symmetrieachse der Verankerungsschale in weiten Grenzen durch in ihrer Länge anpassbare Abstandselemente frei festgelegt werden. Dadurch wird eine optimale Rekonstruktion des Drehzentrums mit grosser Genauigkeit möglich. Die weitgehend freie Positionierbarkeit der Innenschale in Bezug auf die Symmetrieachse ist durch die längsovale halboffene Form der Verankerungsschale und durch zwei, mit der Verankerungsschale verbundene Abstandselemente, die unterhalb der Äquatoriallinie der Schale gegenüber dem fehlenden Quadranten angeordnet sind, möglich. Diese Abstandselemente können in ihrer Länge variiert werden. Das kann zum Beispiel durch Kürzung der Elemente mittels eines geeigneten Werkzeugs an vorgegebenen Sollbruchstellen bewerkstelligt werden. Aber auch eine Verlängerung der Abstandselemente ist durch zusätzliche Einrichtungen, wie aufsteckbare Kappen, möglich. In einer besonders bevorzugen Variante sind die Abstandselemente lösbar mit der Verankerungsschale, z.B. durch eine einfache Steck- oder Schraubverbindung, verbunden. In diesem Fall besteht die Möglichkeit, dem Operateur eine ganze Reihe von Abstandselementen unterschiedlicher Länge zur Verfügung zu stellen, aus denen dann ein Abstandselement in der gewünschten Länge auswählbar ist.

Im folgenden wird die Erfindung an Hand der Zeichnung näher erläutert. Es zeigen in schematischer Darstellung:
- Fig. 1: Ein Ausführungsbeispiel einer äusseren Verankerungsschale mit Abstandselementen und Führungsrippen,

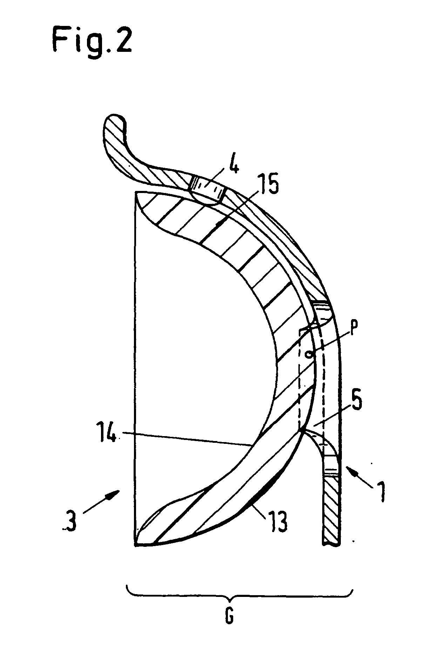

- Fig. 2: eine erfindungsgemässe Gelenkpfanne mit äusserer Verankerungsschale und Innenschale,
- Fig. 3: ein in seiner Länge anpassbares Abstandselement.

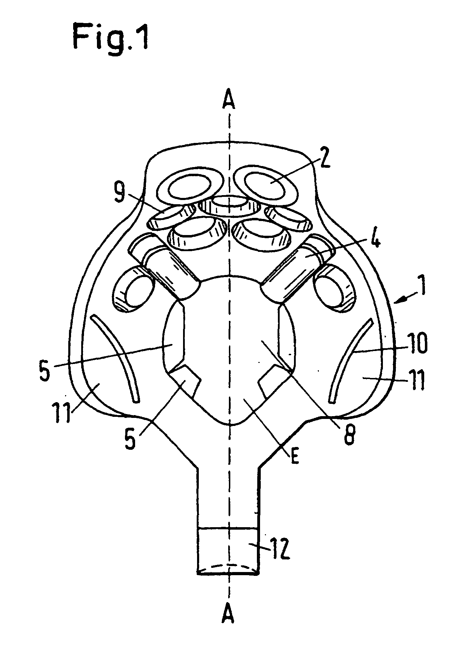

Fig. 1 zeigt in einer schematischen Darstellung eine beispielsweise aus Titan oder einer Titanlegierung bestehende äussere Verankerungsschale 1 zur Fixierung in einer präparierten Knochenhöhle, z.B. in einem Acetabelum. Die Schalenfläche der Verankerungsschale 1 ist in ihrer Grundform oval oder annähernd halbkugelförmig ausgeführt, wobei in einem Quadranten ein sektorförmiger Abschnitt fehlt. Im Polbereich fehlt ein kugelflächenartiger Abschnitt, so dass die Verankerungsschale 1 in diesem Bereich eine Öffnung 8 aufweist, die eine Ebene E definiert. In der Schalenfläche, die vorzugsweise eine Wandstärke von 1 bis 3 mm aufweist, sind Verankerungshilfen 2 zur Primärverankerung der Verankerungsschale 1 mittels nicht gezeigter Befestigungselemente, wie zum Beispiel Knochenschrauben, vorgesehen. Darüber hinaus existieren Öffnungen 9, durch die Knochenzement in den Zwischenraum zwischen Verankerungsschale 1 und Knochengewebe eingebracht werden kann. Ausserdem ist durch die Öffnung 9 der Abstand der Verankerungsschale 1 zum Knochengewebe bestimmbar. An einem Rand der Verankerungsschale 1 befindet sich ein als Sollbruchlinie ausgebildeter Schlitz 10, der es gestattet, von der Verankerungsschale 1 einen peripheren Teil 11 zu entfernen. Je nachdem welcher periphere Teile 11 entfernt ist, kann die künstliche Gelenkpfanne G z.B. in einer linken Körperhälfte des Patienten oder in einer rechten Körperhälfte eingepflanzt werden. Weiterhin weist die Verankerungsschale 1 am Rand der Öffnung 8 Führungsrippen 5 auf, die die Führung der Innenschale 3 entlang einer Achse A - A, die den Bereich des fehlenden sektorförmigen Quadranten schneidet, erlaubt. Die Verbindungsstelle zwischen Führungsrippe 5 und dem Rand der Öffnung 8 ist als Sollbiegelinie ausgebildet. Damit ist durch Biegen der Führungsrippe 5 in vorgegebene Richtungen der senkrechte Abstand, den die Innenschale 3 in Bezug auf die Ebene E einnimmt, einstellbar. In Richtung A - A ist die Verankerungsschale 1 vorzugsweise längsoval ausgeführt, wobei sich im Bereich des fehlenden sektorförmigen Quadranten in Richtung von A - A eine Lasche 12 erstreckt, die biegbar ausgeführt ist und als zusätzliche Verankerungshilfe der Verankerungsschale 1 im angrenzenden Knochengewebe geeignet ist.

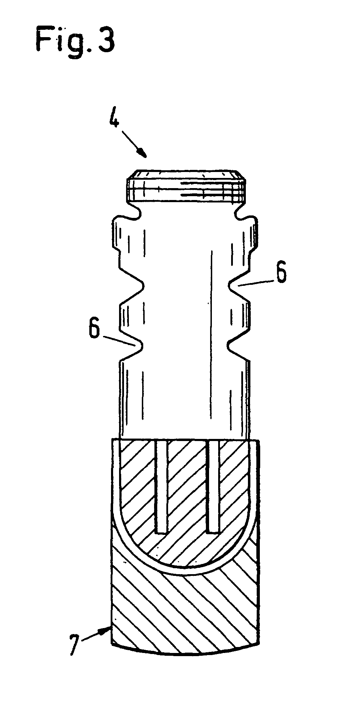

Die Verankerungsschale 1 weist weiterhin mindestens ein Abstandselement 4 auf, das die Positionierung der Innenschale 3 entlang der Linie A - A in Bezug auf die Verankerungsschale 1 erlaubt. Vorzugsweise weist die Verankerungsschale 1 zwei stabförmige Abstandselemente 4 auf, die in ihrer Länge unabhängig voneinander einstellbar sind. Die Abstandselemente 4 können aus einem körperverträglichen Metall oder einer entsprechenden Metallegierung, einem körperverträglichen Kunststoff, aus ausgehärtetem Knochenzement oder einem anderen geeigneten Material bestehen. Das Abstandselement 4 kann dabei lösbar, z.B. mittels einer Verschraubung, einem Bajonettverschluss oder einem anderen geeigneten Befestigungsmittel mit der Verankerungsschale 1 verbunden sein. In einer einfachen Ausführungsvariante ist das Abstandselement 4 steckbar ausgeführt, so dass es z.B. in einem Pressitz in einer Öffnung oder auf einem Zapfen lösbar mit der Verankerungsschale 1 verbunden ist. Dann kann in gewissen Grenzen die wirksame Länge des Abstandselements 4 auch durch Variation der Einbringtiefe des Abstandselements 4 in die Verankerungsschale 1 auf ein vorgebbares Mass einstellbar sein. Wenn das Abstandselement 4 lösbar mit der Verankerungsschale 1 verbunden ist, besteht auch die Möglichkeit, dem Operateur eine ganze Reihe von Abstandselementen 4 unterschiedlicher Länge zur Verfügung zu stellen, aus denen dann ein Abstandselement 4 in der gewünschten Länge auswählbar ist. Ein umständliches Verlängern oder Kürzen eines bereits eingesetzten Abstandselementes 4 entfällt dann. Denkbar ist selbstverständlich auch, dass das Abstandselement 4 nicht-lösbar mit der Verankerungsschale 1 verbunden ist. Dabei ist sowohl eine Verlängerung des Abstandselements 4 durch eine zusätzliche Einrichtung 7 auf ein vorgebbares Mass möglich, als auch eine Reduzierung der Länge. Die Einrichtung 7 zur Verlängerung des Abstandselements 4 kann, wie in Fig. 3 gezeigt, z.B. als längsgeschlitzte Kappe ausgebildet sein, die auf ein zu kurzes Abstandselement 4 aufsteckbar ist. Denkbar ist selbstverständlich auch, dass das Abstandselement 4 eine radiale Bohrung besitzt, die zur Aufnahme eines Fixierungsdorns geeignet ist, der sich an der Einrichtung 7 befinden kann. Die Einrichtung 7 zur Verlängerung des Abstandselements 4 kann auch mittels einer Verschraubung oder auf andere Weise erfolgen. Zur Kürzung des Abstandselements 4 auf ein vorgebbares Mass weist das Abstandselement 4 entlang eines Umfangs mindestens einen Bereich 6 auf, der als Sollbruchstelle ausgebildet ist, so dass der Operateur, z.B. mittels eines Werkzeugs, die Länge des Abstandselements 4 anpassen kann. Schliesslich ist sowohl durch die Abstandselemente 4, als auch durch die Führungsrippen 5 gewährleistet , dass die Innenschale 3 zur Verankerungsschale 1 so auf Distanz gehalten wird, dass zwischen den beiden Schalen ein Zementbett von entsprechender Dicke besteht.

Die Innenschale 3 dient zur Aufnahme eines Gelenkkopfes und kann ein- oder mehrteilig ausgeführt sein. Die die Innenschale 3 aufbauenden Materialien können z.B. körperverträgliche Kobaltlegierungen, körperverträgliche Kunststoffe, wie beispielsweise Polyethylen oder keramische Stoffe umfassen. Die Innenschale 3 weist einen annähernd halbkugelförmigen Innenraum zur Aufnahme eines Gelenkkopfes auf. Die Aussenschale 13 der Innenschale 3 ist vorteilhaft halbkugelförmig ausgebildet, kann jedoch auch z.B. bezüglich einer Achse oval oder halboval ausgebildet sein. Bei einer mehrteiligen Innenschale 3 kann die Aussenschale 13 aus Titan oder einer Titanlegierung bestehen. Insbesondere wenn die Lauffläche 14 einer mehrteiligen Innenschale 3 aus einem anderen Metall, z.B. aus einer Kobaltlegierung, besteht, als die Aussenschale 13, ist die Lauffläche 14 von der Aussenschale 13 durch einen Kunststoffkörper 15 getrennt, um elektrochemische Reaktionen zu vermeiden. Die Lauffläche 14 kann aber auch aus einer geeigneten Keramik aufgebaut sein, wobei der Kunststoffkörper in diesem Fall ganz fehlen kann.

Die erfindungsgemässe künstliche Gelenkpfanne stellt dadurch, dass die Innenschale, die zur Aufnahme des Gelenkkopfes dient, auch noch nach der Fixierung der Verankerungsschale in der präparierten Knochenhöhlung in weiten Grenzen frei positionierbar ist, einen erheblichen Fortschritt zum Stand der Technik dar. Die Verankerungsschale weist Abstandselemente auf, die in ihrer Länge frei einstellbar ausgeführt sind, so dass durch die Innenschale - deren Position im implantierten Zustand die Lage des Gelenkkopfes definiert - eine hervorragende Rekonstruktion des Drehzentrums des Gelenkes ermöglicht wird. Durch zusätzliche Führungsrippen, die in der Nähe des Polbereichs der Verankerungsschale angeordnet sein können, ist die Position der Innenschale zusätzlich justierbar. Darüber hinaus gewährleisten sowohl die Abstandselemente, als auch die Führungsrippen, dass die Innenschale zur Verankerungsschale so auf Distanz gehalten wird, dass zwischen den beiden Schalen ein Zementbett von entsprechender Dicke besteht.

## Patentansprüche

1. Künstliche Gelenkpfanne umfassend eine äussere Verankerungsschale (1) mit Verankerungshilfen (2) zur Fixierung der Verankerungsschale (1) mittels Befestigungselementen in einer Knochenhöhlung und eine Innenschale (3) zur Aufnahme eines Gelenkkopfes, wobei an der Verankerungsschale (1) mindestens ein Abstandselement (4) so angeordnet ist, dass die Innenschale (3) in der Verankerungsschale (1) durch das Abstandselement (4) in Bezug auf die Verankerungsschale (1) positionierbar ist, **dadurch gekennzeichnet, dass** die Länge des Abstandselementes (4) auf ein vorgebbares Mass anpassbar und ein Lagerflächenmittelpunkt (P) der Innenschale (3) entlang einer vorgebbaren Kurve verschiebbar ist,
indem entweder
an dem Abstandselement (4) und/oder der Verankerungsschale (1) Mittel (6, 7) zur Änderung der wirksamen Länge des Abstandselementes vorgesehen sind,
oder
das Abstandselement (4) als separates Bauteil ausgebildet und mit der Verankerungsschale verbunden ist, wobei eine Reihe von Abstandselementen unterschiedlicher Länge zur Auswahl des Abstandselementes (4) vorgesehen ist.

2. Künstliche Gelenkpfanne nach Anspruch 1, welche mindestens zwei stabförmige Abstandselemente (4) zur Positionierung der Innenschale (3) aufweist, wobei die Länge der Abstandselemente (4) unabhängig voneinander auf vorgebbare Masse anpassbar sind.

3. Künstliche Gelenkpfanne nach einem der vorhergehenden Ansprüche, bei welcher die äussere Verankerungsschale (1) Führungsrippen (5) zur Positionierung der Innenschale (3) aufweist.

4. Künstliche Gelenkpfanne nach einem der vorhergehenden Ansprüche, bei welcher die Abstandselemente (4) wirkfest mit der Verankerungsschale (1) verbunden sind.

5. Künstliche Gelenkpfanne nach einem der Ansprüche 1 bis 3, bei welcher die Abstandselemente (4) lösbar mit der Verankerungsschale (1) verbunden sind.

6. Künstliche Gelenkpfanne nach einem der vorhergehenden Ansprüche, bei welcher das Abstandselement (4) entlang eines Umfangs mindestens einen Bereich (6) aufweist, der so ausgebildet ist, dass die Länge des Abstandselements (4) entlang dieses Bereiches (6) auf das vorgebbare Mass anpassbar ist.

7. Künstliche Gelenkpfanne nach Anspruch 5, bei welcher die Länge des Abstandselements (4) durch Variation der Einbringtiefe in die Verankerungsschale (1) einstellbar ist.

8. Künstliche Gelenkpfanne nach einem der vorhergehenden Ansprüche, bei welcher das Abstandselement (4) durch eine zusätzliche, das Abstandselement verlängernde Einrichtung (7) auf ein vorgebbares Mass anpassbar ist.

9. Künstliche Gelenkpfanne nach einem der vorhergehenden Ansprüche, bei welcher die Verankerungsschale (1) bezüglich einer Achse A - A längsoval ausgeformt ist.

10. Künstliche Gelenkpfanne nach einem der vorhergehenden Ansprüche, bei welcher die Verankerungsschale (1) eine Wandstärke von 1 bis 3 mm aufweist.

11. Künstliche Gelenkpfanne nach einem der vorhergehenden Ansprüche, bei welcher die Innenschale (3) einteilig aufgebaut ist und die die Innenschale (3) aufbauenden Materialien eine Kobaltlegierung, einen körperverträglichen Kunststoff, wie beispielsweise Polyethylen oder keramische Stoffe umfassen.

12. Künstliche Gelenkpfanne nach einem der Ansprüche 1 bis 10, bei welcher die Innenschale (3) mehrteilig ausgeführt ist und die die Innenschale (3) aufbauenden Materialien Titan, eine Titanlegierung, eine Kobaltlegierung, einen körperverträglichen Kunststoff, wie beispielsweise Polyethylen oder keramische Stoffe umfassen.

13. Künstliche Gelenkpfanne nach einem der vorhergehenden Ansprüche, bei welcher das Abstandselement (4) aus Titan, einer Titanlegierung, einer Kobaltlegierung, einem körperverträglichen Kunststoff, wie beispielsweise Polyethylen, einem keramischen Material oder aus ausgehärtetem Knochenzement (Polymethylenmethylacrylat) besteht.

## Claims

1. An artificial joint socket including an outer anchor shell (1) having anchor aids (2) for fixing the anchor shell (1) in a bone cavity by means of fastening elements and having an inner shell (3) for receiving a condyle, wherein at least one spacing element (4) is arranged at the anchor shell (1) such that the inner shell (3) can be positioned in the anchor shell (1) by the spacing element (4) with respect to the anchor shell (1), **characterised in that** the length of the spacing element (4) can be adapted to a pre-settable dimension and a bearing surface centre (P) of the inner shell (3) is displaceable along a pre-settable curve, **in that** either means (6, 7) for changing the effective length of the spacing element are provided at the spacing element (4) and/or at the anchor shell (1) or that the spacing element (4) is made as a separate component and is connected to the anchor shell, with a series of spacing elements of different length being provided for the selection of the spacing element (4).

2. An artificial joint socket in accordance with claim 1, which has at least two bar-shaped spacing elements (4) for positioning the inner shell (3), wherein the lengths of the spacing elements (4) are adaptable to pre-settable dimensions independently of one another.

3. An artificial joint socket in accordance with any one of the preceding claims, wherein the outer anchor shell (1) has guide ribs (5) for positioning the inner shell (3).

4. An artificial joint socket in accordance with any one of the preceding claims, wherein the spacing elements (4) are effectively fixedly connected to the anchor shell (1).

5. An artificial joint socket in accordance with any one of claims 1 to 3, wherein the spacing elements (4) are releasably connected to the anchor shell (1).

6. An artificial joint socket in accordance with any one of claims 1 to 5, wherein the spacing element (4) has at least one region (6) along a periphery which is designed such that the length of the spacing element (4) can be adapted to the pre-settable dimension along this region (6).

7. An artificial joint socket in accordance with claim 5, wherein the length of the spacing element (4) can be set by a variation of the insertion depth into the anchor shell (1).

8. An artificial joint socket in accordance with any one of the preceding claims, wherein the spacing element (4) is adaptable to a pre-settable dimension by an additional device (7).

9. An artificial joint socket in accordance with any one of the preceding claims, wherein the anchor shell (1) has an elongate oval shape with respect to an axis A - A.

10. An artificial joint socket in accordance with any one of the preceding claims, wherein the anchor shell (1) has a wall thickness of 1 to 3 mm.

11. An artificial joint socket in accordance with any one of the preceding claims, wherein the inner shell (3) is made of one piece and the materials making up the inner shell (3) include a cobalt alloy, a biocompatible plastic such as polyethylene, or ceramic materials.

12. An artificial joint socket in accordance with any one of claims 1 to 10, wherein the inner shell (3) is made of a multitude of parts and the materials making up the inner shell (3) include titanium, a titanium alloy, a cobalt alloy, a biocompatible plastic such as polyethylene, or ceramic materials.

13. An artificial joint socket in accordance with any one of the preceding claims, wherein the spacing element (4) consists of titanium, a titanium alloy, a cobalt alloy, a biocompatible plastic such as polyethylene, a ceramic material or a hardened bone cement (polymethylene methyl acrylate).

## Revendications

1. Cotyle artificiel comprenant une coquille extérieure d'ancrage (1) munie d'auxiliaires d'ancrage (2) pour verrouiller ladite coquille d'ancrage (1) à demeure dans une dépouille osseuse, au moyen d'éléments de fixation, et une coquille intérieure (3) destinée à recevoir une tête d'articulation, au moins un élément d'espacement (4) étant placé sur la coquille d'ancrage (1) de façon que la coquille intérieure (3) puisse être positionnée dans la coquille d'ancrage (1), par l'intermédiaire de l'élément d'espacement (4), vis-à-vis de ladite coquille d'ancrage (1), **caractérisé en ce que** la longueur de l'élément d'espacement (4) peut être adaptée à une cote pouvant être préétablie, et un point central (P) de la surface de portée de la coquille intérieure (3) peut être déplacé le long d'une courbe pouvant être préétablie,
du fait que
des moyens (6, 7) sont prévus sur l'élément d'espacement (4) et/ou sur la coquille d'ancrage (1), pour modifier la longueur efficace dudit élément d'espacement,
ou que
ledit élément d'espacement (4) est réalisé sous la forme d'une pièce structurelle distincte et est relié à la coquille d'ancrage, une rangée d'éléments d'espacement de longueurs différentes étant prévue pour sélectionner l'élément d'espacement (4).

2. Cotyle artificiel selon la revendication 1, comportant au moins deux éléments d'espacement (4) en forme de barrettes, en vue du positionnement de la coquille intérieure (3), les longueurs desdits éléments d'espacement (4) pouvant être adaptées, indépendamment l'une de l'autre, à des cotes pouvant être préétablies.

3. Cotyle artificiel selon l'une des revendications précédentes, dans lequel la coquille extérieure d'ancrage (1) présente des nervures de guidage (5) en vue du positionnement de la coquille intérieure (3).

4. Cotyle artificiel selon l'une des revendications précédentes, dans lequel les éléments d'espacement (4) sont en liaison opérante fixe avec la coquille d'ancrage (1).

5. Cotyle artificiel selon l'une des revendications 1 à 3, dans lequel les éléments d'espacement (4) sont reliés de manière amovible à la coquille d'ancrage (1).

6. Cotyle artificiel selon l'une des revendications précédentes, dans lequel l'élément d'espacement (4) présente, le long d'un pourtour, au moins une région (6) réalisée de telle sorte que la longueur dudit élément d'espacement (4) puisse être adaptée, le long de cette région (6), à la cote pouvant être préétablie.

7. Cotyle artificiel selon la revendication 5, dans lequel la longueur de l'élément d'espacement (4) est réglable en faisant varier la profondeur de pénétration dans la coquille d'ancrage (1).

8. Cotyle artificiel selon l'une des revendications précédentes, dans lequel l'élément d'espacement (4) peut être adapté à une cote, pouvant être préétablie, par l'intermédiaire d'un dispositif additionnel (7) prolongeant ledit élément d'espacement.

9. Cotyle artificiel selon l'une des revendications précédentes, dans lequel la coquille d'ancrage (1) est de configuration ovalisée par rapport à un axe A-A.

10. Cotyle artificiel selon l'une des revendications précédentes, dans lequel la coquille d'ancrage (1) possède une épaisseur de paroi de 1 à 3 mm.

11. Cotyle artificiel selon l'une des revendications précédentes, dans lequel la coquille intérieure (3) est d'un agencement structurel monobloc, et les matériaux constituant ladite coquille intérieure (3) englobent un alliage au cobalt, une matière plastique biologiquement compatible, comme du polyéthylène par exemple, ou des substances céramiques.

12. Cotyle artificiel selon l'une des revendications 1 à 10, dans lequel la coquille intérieure (3) est réalisée en plusieurs parties, et les matériaux constituant ladite coquille intérieure (3) englobent du titane, un alliage au titane, un alliage au cobalt, une matière plastique biologiquement compatible, comme du polyéthylène par exemple, ou des substances céramiques.

13. Cotyle artificiel selon l'une des revendications précédentes, dans lequel l'élément d'espacement (4) consiste en du titane, en un alliage au titane, en un alliage au cobalt, en une matière plastique biologiquement compatible telle que du polyéthylène par exemple, en un matériau céramique ou en de l'ostéociment durci (polyméthylène-méthylacrylate).
